# EUROPEAN PATENT APPLICATION

(11) **EP 4 755 202 A2**
(43) Date of publication of application: **10.06.2026**
(21) Application number: 26173878.5
(22) Date of filing: 03.11.2022
(51) Int. Cl.: A23L 33/105

(54) **COCOA PLANT CELL CULTURE PROCESSING METHODS AND PRODUCTS OBTAINED BY THE SAME**

(30) Priority: 03.11.2021 EP 21206180
(62) Divisional of application: 22817532.9
(71) Applicant: ZHAW - Zürcher Hochschule für Angewandte Wissenschaften, 8820 Wädenswil (CH)
(72) Inventor: Eibl, Regine, 8820 Wädenswil (CH); Eibl, Dieter, 8820 Wädenswil (CH); Hühn, Tilo, 8820 Wädenswil (CH)
(74) Representative: Grünecker Patent- und Rechtsanwälte PartG mbB

(57) **Abstract**

Method of processing cocoa plant cell cultures, comprising the induction of callus from cocoa bean explants or enzymatic disaggregation of cocoa bean cells, growing cocoa bean cells in the suspension cell culture for a time and under conditions sufficient to produce a biomass comprising secondary metabolites; and separating the biomass from the suspension cell culture to provide a liquid phase and in vitro-manufactured cocoa powder in a bioreactor selected from a stirred tank bioreactor, an orbitally shaken bioreactor, a bubble column bioreactor or an air-lift bioreactor. The method enables cocoa powder to be manufactured in *vitro* effectively in the quantity and quality required to compete with conventional cocoa powder processing. In addition, methods of manufacturing chocolate or chocolate-like products using said in vitro-manufactured cocoa powder, uses of the latter, and related products are described.

## Description

### FIELD OF INVENTION

This invention relates to methods and/or techniques for the *in vitro*-production of cocoa powder on a large scale and its further processing to chocolate, chocolate-like products or extracts based on cocoa plant cell cultures.

In certain embodiments, this invention relates to use of said *in vitro*-manufactured extracts as food, food additive or in cosmetic or pharmaceutical preparations.

In addition, chocolate and chocolate-like products obtained by said methods and/or techniques are disclosed.

### BACKGROUND OF THE INVENTION

In the recent decades, cacao plants (*Theobroma cacao L.*, Sterculiaceae) have been increasingly chased out of their natural habitats by climate change and fungal diseases. Such pests and diseases have unpredictable effects on yearly cocoa production volumes, and unexpected changes in weather conditions further contribute to the volatile output. Moreover, conventionally cultivated cocoa is subject to seasonal quality fluctuations resulting from post-harvesting treatment. Various studies have therefore reported that it will become increasingly difficult in the next years and decades to meet the growing demand for cocoa products in sufficient quantity and quality.

Production of cocoa powder based on plant cell culture technology would mitigate many of the problems associated with traditional cocoa powder production. For example, cocoa cell culture cultivation may be performed in a bioreactor under controlled Good Laboratory Practice (GLP) or even Good Manufacturing Practice (GMP) conditions, which guarantees a consistent and locally and seasonally independent quality of the resulting cocoa powder. In addition, the use of herbicides, pesticides or genetical manipulation to enhance the resistance of cocoa plants against diseases may be entirely avoided. In addition, less agricultural space would be required and deforestation pressure could be reduced, while the necessary energy consumption and emissions would be lower compared to conventional agriculture. Moreover, the competition based on biotechnological production could provide an incentive for conventional cocoa farmers and chocolate manufacturers to improve the transparency of the supply chain, to mitigate problematic and/or non-ecological cultivation practices, and to promote fair trade without child labor practices in the countries of origin.

Plant cell-based cellular agriculture using plant cell cultures to manufacture high-value food supplements, such as Ginseng triterpene saponins, has been practiced for a long time (see, e.g. J. Wu, J.J. Zhong; J. Biotechnol. 1999, 68(2-3), 89-99).

Also, US 2012/0021080 A1 and US 2012/0142105 A1 disclose preparation methods of cocoa polyphenol compositions and cocoa oligomeric procyanidins based on callus cell cultures from immature *Theobroma* sp. floral explants. A. M. Rúa et al., Bioprocess and Biosystems Engineering 2017, 40(10), 1479-1492 discloses an analysis of lipid profiles in cacao cell suspensions. CN 102687838 A discloses a method for preparation of cocoa extracts, which is based on enzymolysis of pulverized cocoa beans with a mixed enzyme composition.

Recent publications further suggest that plant cell cultures or their extracts may themselves be used as foodstuffs (see, e.g., E. Nordlund et al., Food Res Int. 2018, 107, 297-305). However, low culture yields and high costs associated with elaborate equipment and its operation and maintenance often impede widespread production of plant cell cultures as food. For example, R. Eibl et al., Applied Microbiology and Biotechnology 2018, 102(20), 8661-8675 discloses the use of a single-use wave-mixed bioreactor for the preparation of sufficient T. *cacao* cell mass to provide a single bar of cell culture chocolate. However, the publication concludes that the need for low-cost bioreactors which are easy to handle and provide sufficient cell mass for food production (even in a three-digit and four-digit g-range) still remains.

In fact, a method based on plant cell culture technology which could enable cocoa powder to be manufactured *in vitro* effectively in the quantity and quality demanded by the food industry is currently not known. In addition, it would also be desirable to provide a method which simultaneously enables a controlled fine-tuning of the sensory properties (e.g. in terms of primary taste profile) and yield of secondary metabolites by adjusting the process parameters and conditions.

Accordingly, the provision of methods and products that overcome the above disadvantages is desirable.

### SUMMARY OF THE INVENTION

The present invention solves this object with the subject matter of the claims as defined herein. The advantages of the present invention will be further explained in detail in the section below and further advantages will become apparent to the skilled artisan upon consideration of the invention disclosure.

Generally speaking, the present invention provides a method of processing cocoa plant cell cultures, comprising the steps of: a1) inducing callus from a cocoa bean explant on callus induction medium and propagating the callus on callus growth medium; b1) selecting a callus clone from the grown callus culture and initiating a suspension cell culture comprising dedifferentiated cells by inoculating the selected callus clone into a suspension cell culture medium; c) growing cocoa bean cells in the suspension cell culture for a time and under conditions sufficient to produce a biomass comprising secondary metabolites; and d) separating the biomass from the suspension cell culture to provide a liquid phase and *in vitro-*manufactured cocoa powder having an acetic acid content of 200 mg per kg of dry weight or less, and optionally further processing said cocoa powder to provide cocoa aroma or a polyphenolic extract; wherein step c) is carried out in a bioreactor selected from a stirred tank bioreactor, an orbitally shaken bioreactor, a bubble column bioreactor or an air-lift bioreactor, preferably a bubble column bioreactor or an air-lift bioreactor.

In an alternative aspect, the present invention provides a method of processing cocoa plant cell cultures, comprising the steps of: a2) incubating a cocoa bean explant in a liquid medium comprising a cell wall disaggregating enzyme, preferably pectinase, to obtain free cocoa bean cells in suspension; b2) subjecting the cocoa bean cells obtained in step a2) to an adaptation step by transferring the same to a liquid medium without the cell wall disaggregating enzyme; c) growing cocoa bean cells in the suspension cell culture for a time and under conditions sufficient to produce a biomass comprising secondary metabolites; and d) separating the biomass from the suspension cell culture to provide *in vitro*-manufactured cocoa powder having an acetic acid content of 200 mg per kg of dry weight or less, and optionally further processing cocoa powder to provide cocoa aroma or a polyphenolic extract; wherein step c) is carried out in a bioreactor selected from a stirred tank bioreactor, an orbitally shaken bioreactor, a bubble column bioreactor or an air-lift bioreactor, preferably a bubble column bioreactor or an air-lift bioreactor.

Advantageously, these methods allow sustainable production of healthy cocoa-based food without herbicides or pesticides at a previously inaccessible large scale, while enabling tailoring of the aromatic profile and yield of secondary metabolites of the thus obtained extracts by controlling aroma and flavor formation through the hydrodynamic parameters under which the bioreactors operate.

In further aspects, the present invention relates to *in vitro*-manufactured extracts selected from any of the liquid phase, cocoa powder, cocoa aroma or the polyphenolic extract obtained by the aforementioned methods, and to the use of said *in vitro*-manufactured extracts according as food, food additive or in cosmetic or pharmaceutical preparations.

In another aspect, the present invention provides a method of manufacturing chocolate or chocolate-like products, comprising the steps of: providing an *in vitro*-manufactured cocoa powder according to the aforementioned methods; optionally subjecting the cocoa powder to an incubation and/or roasting step; mixing the cocoa powder with cocoa butter and optional additives selected from emulsifiers, aroma, milk solids, sugars and/or cocoa pulp; and conching said mixture.

In a further aspect, the present invention relates to chocolate or chocolate-like products obtained by the latter method. It has been found that the chocolate or chocolate-like products exhibit a more complex aromatic profile, less bitter and/or sour taste in combination with lower alkaloid content (especially theobromine and caffeine) and/or higher antioxidant yield when compared to traditionally manufactured chocolate or chocolate-like products.

### BRIEF DESCRIPTION OF THE DRAWINGS

FIG. 1 schematically illustrates exemplary methods of processing cocoa plant cell structures according to the first and second embodiments of the present invention.
FIG. 2 schematically illustrates an exemplary process of manufacturing chocolate or chocolate-like products according to the fifth embodiment of the present invention.
FIG. 3 shows the uptake of sugars sucrose (Suc), fructose (Fruc) and glucose (Gluc) during the cultivation stage.
FIG. 4 shows the results of the sensory analysis performed on an exemplary cell culture chocolate.
FIG. 5 shows the results of a sensory analysis performed on exemplary cell culture chocolates with a focus on appearance, texture and basic flavors.
FIG. 6 shows the results of the sensory analysis performed on exemplary cell culture chocolates with a focus on specific aromas.

### DETAILED DESCRIPTION OF THE INVENTION

For a more complete understanding of the present invention, reference is now made to the following description of the illustrative embodiments thereof:

### Methods of Processing Cocoa Plant Cell Cultures

In a first embodiment, the present invention relates to a method of processing cocoa plant cell cultures, comprising the steps of: a1) inducing callus from a cocoa bean explant on callus induction medium and propagating the callus on callus growth medium; b1) selecting a callus clone from the grown callus culture and initiating a suspension cell culture comprising dedifferentiated cells by inoculating the selected callus clone into a suspension cell culture medium; c) growing cocoa bean cells in the suspension cell culture for a time and under conditions sufficient to produce a biomass comprising secondary metabolites; and d) separating the biomass from the suspension cell culture to provide a liquid phase and *in vitro-*manufactured cocoa powder having an acetic acid content of 200 mg per kg of dry weight or less, and optionally further processing said cocoa powder to provide cocoa aroma or a polyphenolic extract; wherein step c) is carried out in a bioreactor selected from a stirred tank bioreactor, an orbitally shaken bioreactor, a bubble column bioreactor or an air-lift bioreactor, preferably a bubble column bioreactor or an air-lift bioreactor.

In contrast to the methods disclosed in US 2012/0021080 A1 and US 2012/0142105 A1, the present invention is not based on the cultivation of callus cell cultures from immature *Theobroma* sp. floral explants, such as petals, sepals, or staminodes, but uses the seeds of cocoa fruits, i.e. the cocoa beans, to establish the production cell line.

In view of a public reluctance to accept genetically modified food sources, the cultivated cell cultures are preferably not genetically modified. Moreover, it is further preferred that any metabolic engineering applied in the present process is performed without genetic modification technology. Beside of the aspiration to preserve the natural state of the cocoa source and to simulate natural biotransformation as closely as possible, such an approach also favourably reduces the regulatory hurdles for commercialization.

It will be understood that the expression "cocoa plant", as used herein, encompasses the genus *Theobroma,* including the species *Theobroma cacao L.*, *Theobroma grandiflorum, Theobroma obovatum, Theobroma speciosum, Theobroma subincanum* and *Theobroma sylvestris.* In a preferred embodiment, the expression "cocoa plant" specifically relates to the species *Theobroma cacao L.*, including its varieties Criollo, Forestero, Trinitario and subvarities of the latter.

While the maturity degree of the cocoa fruits is not specifically limited, the use of fruits identified as ripe by colour and manual inspection is preferred.

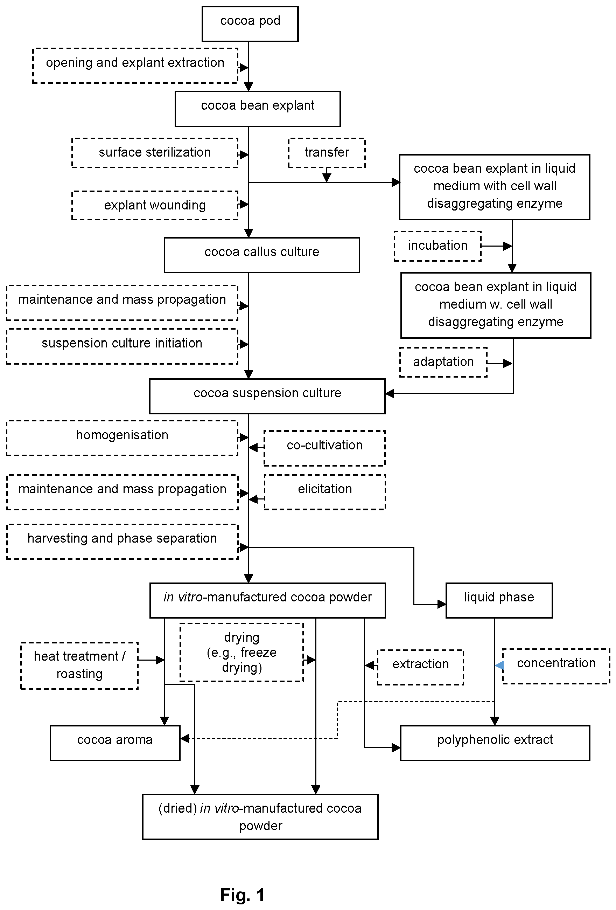

An exemplary method according to the first embodiment or second embodiment (which will be explained below) of the invention is illustrated in Fig. 1. However, it is understood that the methods are not limited to the depicted process.

The subject process typically begins with opening or cutting open a washed and/or surface-sterilized cocoa pod and extracting cocoa bean(s) from the pod under aseptic conditions, which is preferably followed by a step of carefully removing the skin of the extracted cocoa beans and sterilizing the thus exposed surface.

The washing and/or surface sterilization steps are not particularly limited as long as they effectively reduce microbial surface contamination, and suitable methods may include chemical sterilization (e.g., with ethanol solution (≥ 70% (v/v)), sodium hypochlorite solution (e.g. 5% (v/v) or the like), washing in sterile water, surface sterilization or pasteurization by means of heat and/or irradiation with ionizing rays (e.g. UV irradiation), ultrasound-based sterilization and combinations thereof.

In step a1), formation of callus, a mass of unorganized, undifferentiated cells is induced by wounding of the cocoa bean explant (e.g. by cutting) and incubating the same onto a callus induction medium.

The callus induction medium is not especially limited and typically represents a semisolid or preferably solid tissue culture medium comprising the ions Ca²⁺, SO₄²⁻, Mg²⁺, PO₄³-, Cl⁻, NO₃⁻ and NH₄⁺. Examples thereof include, but are not limited to Murashige and Skoog (MS) medium (Murashige and Skoog, Physiol. Plant 1962, 15, 473-497), WPM medium (Lloyd and McCown, Int. Plant Prop. Soc. Proc. 1980, 30, 421), DKW basal medium (Driver and Kuniyuki, Hortsci. 1984, 19, 507-509), Gamborg B5 medium (Gamborg, Miller Ojima, Exp. Cell res. 1968, 50, 151) or similar plant tissue culture (PTC) media and modifications thereof.

The callus induction medium typically further contains carbohydrates (such as glucose, sucrose or saccharose) as a carbon source. In preferred embodiments, the concentration of the carbon source in the (fresh) callus induction medium is at least 10 g/l, more preferably higher than 15 g/l, further preferably between 18 and 40 g/L and especially preferably between 20 and 30 g/l.

The cocoa bean explants are preferably cultured on the callus induction medium at temperatures of 25 ± 5° C for a period of 5 to 35 days, such as from 10 to 20 days, preferably under dark growth conditions.

Subsequently, the induced callus is transferred and propagated on callus growth medium.

The callus growth medium may contain higher levels of carbohydrates (such as glucose, sucrose or saccharose) as a carbon source than the callus induction medium to support growth and production of secondary metabolites. In preferred embodiments, the concentration of the carbon source in the fresh callus growth medium is at least 25 g/l, more preferably higher than 30 g/l, further preferably at least 40 g/l and especially preferably between 45 and 75 g/l.

In preferred embodiments, the callus induction and/or callus growth media are further supplemented with one or more vitamins (e.g. Vitamin B5) and/or one or more growth regulators (e.g., sterile coconut water, a cytokinin (such as thidiazuron, zeatin or kinetin), gibberellin (e.g., gibberellic acid) or an auxin (e.g., 2,4-dichlorophenoxyacetic acid (2,4-D), indoleacetic acid (IAA) or indolebutyric acid (IBA)) or preferably a combination thereof. Phytohormones present in or extracted from meristem plant cells, preferably those of *Theobroma cacao,* and especially preferably apical meristem (shoot apical meristem and/or root apical meristem) cells of *Theobroma cacao* may be preferably used, which also enable induction and growth control without addition of chemically synthesized growth regulators. In the case of the growth medium, it is important to appropriately select the growth regulators so as to avoid any negative effects on the consumer during the later application of the cell culture. For instance, for the manufacture of a food product, it is important to select food-compliant growth regulators for the callus growth medium.

Callus cultures may be preferably maintained in fresh callus growth medium by periodically transferring at least a portion of the callus to a fresh medium under aseptic conditions, typically in a biomass/medium weight ratio of between 20:80 to 40:60 (subcultivation). Suitable subcultivaton intervals (typically around 3 to 4 weeks) may be determined by the skilled artisan depending on the growth performance and the decrease of carbohydrate and nitrogen concentration in the medium (e.g. by periodical sampling and HPLC analysis).

Notably, step a1) may include further substeps performed in series (e.g. subcultivation, transfer to multiple types of callus induction media and/or multiple types of callus growth media) or in parallel (e.g. combination of different induced calli for the growth phase).

Once sufficient callus mass has been established, a well-growing and producing callus line is selected and a suspension cell culture comprising dedifferentiated cells is initiated by inoculating the selected callus mass into a liquid medium supporting cocoa cell growth and the production of secondary metabolites of interest in step b1).

Optionally, the selected calli may be first transferred or pooled into shake flasks containing liquid culture medium and shaken to promote homogenisation during step b1) or before being transferred to a bioreactor to step c).

The suspension cell culture medium used in step b1) and/or c) is not particularly limited and may be a liquid variant of the callus growth medium described above.

In general, it is preferred that the suspension cell culture medium at least comprises a plurality of mineral salts, further preferably in combination at least one or more growth regulators (e.g., phytohormones) and at least one carbohydrate as carbon source.

Preferably, the concentration of a carbohydrate (such as glucose, sucrose or saccharose) in the fresh suspension cell culture medium is at least 25 g/l, more preferably at least 30 g/l, further preferably at least 40 g/l and especially preferably between 45 and 75 g/l. Working within these ranges was shown to enable an unexpectedly high increase of biomass growth.

Suspension cultures may subcultivated by periodically transferring at least a portion of the cultured cells to a fresh medium under aseptic conditions, typically in a biomass/medium weight ratio of between 20:80 to 40:60. Typical subcultivaton intervals range from around 14 to 21 days, and may be suitably adjusted by the skilled artisan upon monitoring the cell growth performance and the decrease of carbohydrate and nitrogen concentration in the suspension medium.

For step c), the suspended cultures are transferred into the bioreactor and grown for a time and under conditions sufficient to produce a biomass comprising secondary metabolites.

According to the present invention, the bioreactor used in step c) is selected from a stirred tank bioreactor, an orbitally shaken bioreactor, a bubble column bioreactor or an air-lift bioreactor, preferably a bubble column bioreactor or an air-lift bioreactor. Notably, although such reactors are commonly known, especially for fermentation purposes (e.g. in beverage production), their successful application for the growth of cocoa suspension cells is all but trivial. For instance, a rigid cell wall and extensive vacuoles are known to make plant cells sensitive to shear stress, so that - contrary to fermentation systems - an upscaling of plant cell culture production via stirred tank bioreactors is often limited and/or hindered by cell death induced by the impeller. In general, several difficulties are typically associated with the exchange and upscaling of bioreactor technology, which mainly involve reduced yield, small growth rates and product release, underexpression of desirable traits and poor culture stability. Advantageously, it has been found that stirred tank bioreactors, orbitally shaken bioreactors, bubble column bioreactors and air-lift bioreactors may be effectively operated at a scale required for cost-effective processing of food and simultaneously enable favourable control of hydrodynamic parameters and aeration conditions to ensure excellent and consistent quality of the *in vitro*-manufactured cocoa powder. In addition, it has been surprisingly found that the use of these reactor types may lead to an improved yield of polyphenolic compounds when compared to wave-mixed bioreactors, for example.

In preferred embodiments, the bioreactor has a volume of at least 0.05 m³, more preferably at least 0.1 m³, further preferably at least 1 m³, and especially preferably at least 5 m³, such as 10 m³ or more, 100 m³ or more, 150 m³ or more, or even 200 m³ or more.

Bubble column bioreactors or air-lift bioreactors are particularly preferred as they may be simultaneously operated at the aforementioned high scales, built with larger working volumes than stirred tank bioreactors, allow oxygen mass transfer at low power input levels, and are inexpensive in terms of investment and maintenance, which is a crucial factor for cost-effective production of plant cell cultures at large scale.

If a stirred tank bioreactor is used, designs using an end-face mechanical seal or a magnetic drive with free levitating impeller are preferably employed, since they are almost maintenance-free and reduce risk of contamination. The type of impeller and its dimensions may be suitably selected by the skilled artisan depending on the mixing time, shear gradient, specific power input and oxygen transfer rate. Multi-stage configurations combining axial and radial flow impellers may also be employed.

In a preferred embodiment, the bioreactor is equipped with an air recirculation system. By operating the bioreactor (especially the airlift and bubble column reactors) so that air bubbles transported through the cell suspensions are recirculated, the oxygen flow rate may be controlled at desirably low levels without imparting the mixing operation, while aromatic volatiles are retained or re-fed in the system, which tends to favourably affect the aroma yield and/or aromatic profile of the obtained cocoa powder. Simultaneously, air recirculation reduces the amount of sterilized air necessary for bioreactor operation and mixing, which may substantially lower the manufacturing costs, especially in large-scale production.

In preferred embodiments, hydrodynamic parameters and aeration conditions of the bioreactor are determined based on the concentration of target secondary metabolites present in the cocoa powder obtained in step d).

In general, the term "secondary metabolites", as used herein, denotes compounds that are not required for the growth or reproduction of the cocoa plant but are produced to confer a selective advantage to the same. While not being limited thereto, exemplary secondary metabolites produced in cocoa beans include xanthines, flavan-3-ols, and oligomeric procyanidins, for example. An overview of secondary metabolites produced during the natural cacao seed development is also disclosed in L. Wang et al., The Plant Journal 2016, 87, 318-332.

In further preferred embodiments, the target secondary metabolites selected to control hydrodynamic parameters and aeration conditions comprise one or more selected from the group of polyphenols (for example, cinnamtannins, procyanidins, epicatechin), vitamins (e.g., vitamin A, vitamin B1, vitamin B2, vitamin B3, vitamin C, and vitamin E), and aromatic compounds (e.g., 1-methyl-1H-pyrrole, ethyl-2-methyl butanoate, 3-methylbutyl acetate, 2-heptanone, hexyl acetate, linaool, benzyl alcohol, 2-phenylethanol, ethyl cinnamate, 2-phenethyl acetate, 2-phenyl-2-butenal and ethyloctanoate). The concentrations of individual target secondary metabolites may be monitored (by methods known in the art, including HPLC-MS or GC-MS) during the cultivation phase and hydrodynamic parameters or aeration conditions may be adjusted if the measured concentration deviates form a predetermined target value.

It is noted that the yield of secondary metabolites is highly sensitive to changes in type, as well as hydrodynamic parameters and aeration conditions of the bioreactor (particularly mixing time, specific power input and/or volumetric oxygen transfer coefficient). Accordingly, their adjustment enables tuning and consistent control of the desired sensory properties (including the aromatic profile) and texture of the resulting *in vitro*-manufactured cocoa powder.

The specific power input is typically adjusted within a range of 50 to 900 W/m³, preferably between 80 and 850 W/m³, and further preferably between 100 and 800 W/m³. It has been surprisingly found that the polyphenol content, aroma and flavor profile and of the resulting cocoa powder may be varied in a simple manner by operating in different specific power input regimes within the above-identified range. For instance, it has been shown that a low specific power input tends to promote the development and content of fruity aromas in the resulting cocoa powder and chocolate. On the other hand, relatively high specific power input shifts the aromatic profile towards intense caramel aromas and a higher bitterness and adstringency.

The volumetric oxygen transfer coefficient is preferably adjusted within a range of 0.3 to 80 h⁻¹, more preferably in a range of from 1 to 75 h⁻¹, especially preferably in a range of 5 to 50 h⁻¹. In case of airlift and bubble column reactors, the volumetric oxygen transfer coefficient may be higher, e.g. in the range of 0.3 to 180 h⁻¹, preferably in a range of 1 to 120 h⁻¹, more preferably in a range of 5 to 100 h⁻¹. However, when operating the latter reactors with an air recirculation system, a range of 0.3 to 80 h⁻¹ may be preferable.

By monitoring the concentration of the abovementioned secondary metabolites, the mixing time may be further adjusted to ensure optimized yield.

Beside of the control of hydrodynamic and aeration parameters, step c) may involve further sub-steps to promote the growth rate, stimulate production of secondary metabolites and control the sensory properties of the resulting cocoa powder.

For instance, one or more homogenisation steps may be incorporated into step c) to remove floating particles and avoid formation of larger cell aggregates. While methods of homogenisation are not particularly limited, gentle homogenisation procedures such as sedimentation and/or filtration are preferred to ensure that the average size of cell aggregates does not exceed 500 µm, which increases the specific growth surface and thus promotes biomass growth velocity without damaging cell components sensitive to heat, pressure and/or mechanical stress.

In addition, multiple-stage cultures may be considered to further promote the development of secondary metabolites in order to account for the tendency that several developmental stages are required for production of the non-growth associated compounds.

In another preferred embodiment, step c) comprises co-cultivating cocoa suspension cells with microorganisms. The term "microorganism", as used herein, is understood to encompass microscopic organisms, which may exist in its single-celled form or a colony of cells, including bacteria and single-cellular fungi or algae. Microorganisms are being preferably selected from one or more species from the group of yeasts, lactic acid bacteria and/or acetic acid bacteria, especially preferably from one or more species from the group of lactic acid bacteria and/or acetic acid bacteria.

Step c) preferably also comprises an elicitation step comprising the addition of one or more abiotic or biotic elicitors to stimulate biosynthesis of secondary metabolites, to reduce the production of undesirable substances and/or to reduce the process time necessary to reach a high product concentration. Further, elicitation may result in the formation of novel compounds. Synergistic effects using multiple simultaneous elicitation treatments or sequential elicitor protocols may also be envisaged.

As examples of biotic elicitors, plant hormones (e.g. salicylic acid and its derivatives and analogs), microorganism-derived elicitors (e.g. microbial pathogens, bacterial extracts, bacteria- and fungi-derived peptides and proteins) and plant cell wall fragments (e.g. chitosan, chinin, oligosaccharins) may be mentioned. Examples of pathogens known to be particularly effective on cocoa plants include, but are not limited to oomycetes (e.g. species of *Phytophthora*) and basidiomycete fungi (e.g. *Moniliophthora perniciosa*)*.*

Abiotic elicitation includes abiotic stress used to elicit the production of defense chemicals (e.g. phytoalexins), e.g. by variation of pressure and/or temperature; addition of natural agents extracted from other plants; addition of chemical compounds, such as AgNO₃, AlCl₃, CaCl₂, CuCl₂, KCI, zinc ions or ozone; addition of pathogen-derived compounds, pesticides, herbicides, fungicides, or bactericides; limitation of sources of phosphorus, nitrogen and/or carbon; water limitation; exposure to high salt concentrations; and exposure to UV-light, microwaves, ultrasonic waves, radiofrequency, infrared radiation, or ionizing radiation.

In a particularly preferred embodiment of abiotic elicitation, growth of the biomass in step c) is enhanced by exposing the suspension cell culture to artificial light having a wavelength in the range of 300 to 700 nm and with an intensity sufficient to promote biomass growth and/or by removal of a fraction of the liquid medium during the growth phase.

It is also preferred that step c) comprises the addition of enzymes, phytohormones or biosynthetic precursors to the medium. Biosynthetic precursors include aroma precursors naturally produced in cocoa beans or aroma precursors produced by another species, or amino acids (such as glutamine, glycine, and serine), for example.

Once a cell culture has reached the desired concentration and/or composition of target secondary metabolites, the cells are harvested and biomass is separated from the suspension cell culture in step d) to provide *in vitro*-manufactured cocoa powder.

The separation method is not particularly limited and may include one or more solid-liquid separation steps, such as filtration, sedimentation, pressing (e.g., screen or screw pressing) decanting or centrifugation under sterile conditions, for example.

In preferred embodiments, the solid phase is rinsed and washed in sterile water to eliminate flavor artifacts originating from the culture medium.

Optionally, the solid phase may be subjected to a grinding step (e.g., to an average particle diameter of 300 µm or less, 200 µm or less, or 100 µm or less) in order to alter the texture of the resulting cocoa powder, preferably in terms of single or multiple wet grinding step(s) in the culture medium before solid-liquid separation or upon adding sterile water or an alternative water-containing liquid to the separated solid phase. At this stage, additional flavors may be introduced with the use of said alternative water-containing liquid, such as coffee, tea, fruit juices, fruit juice concentrates, milk or the like. Grinding may be performed by using disc mills (e.g. a perforated disc mill), colloid mills (e.g. toothed colloid mills), or corundum stone mills, for example.

The thus obtained cocoa powder is preferably subjected to drying prior to packaging, storage and/or further processing, e.g. by drum drying, rotary drying or freeze-drying, among which freeze-drying is especially preferred.

Unlike known methods for extracting cocoa polyphenol compositions and cocoa oligomeric procyanidins based on suspension cell cultures, the present method provides an *in vitro*-manufactured cocoa powder ready to be directly processed into food products and hence does not require extraction steps which involve the use of undesirable organic solvents (such as hexane, for example).

However, solvent extraction may be employed to provide a polyphenolic concentrate from the solid phase or cocoa powder, respectively. In this case, it is preferred to use aqueous organic solvents comprising ethanol, methanol, acetone, isopropyl alcohol, ethylacetate or combinations thereof, optionally in combination with an acid (e.g., ascorbic acid). The solvent may be then removed by one or more concentration steps to provide polyphenolic extract. In order to minimize heat-induced degradation of polyphenols, concentration methods at low temperatures (≤ 45°C) and reduced pressure are preferred.

The cocoa powder may be subjected to a step of drying and/or roasting, during which cocoa aroma may be separated. Gentle drying and roasting is preferably performed at a temperature of between 50 and 100°C, more preferably between 55 and 75°C under reduced pressure, in order to allow roasted flavors and other aromatics to be collected.

The liquid phase obtained after solid-liquid separation may also contain desirable water-soluble flavors and especially antioxidants, which may be optionally recovered by subjecting the liquid phase to a concentration step. Enhancement of cocoa flavors may be achieved using reverse flow distillation (i.e., to separate flavor compounds and water), for example, preferably under low pressure (less than 300 mbar) and room temperature in order to minimize the thermal load. For an improved extraction yield of water-soluble flavors and antioxidants, it is preferred to carry out the aforementioned wet-grinding step to an average particle diameter of 200 µm or less and further preferably to 100 µm or less, before solid-liquid separation. If necessary, a pasteurisation or sterilisation step may be carried out according to methods known in the art to prevent microorganism spoilage/propagation, e.g. by application of heat, irradiation, chemical sterilisation, and micro-, ultra- or nanofiltration, for example. The pasteurisation process may be carried out in batch or continuous operation, e.g. by the use of HTST (high temperature short time) heat exchangers. In further concentration phase, evaporation of excessive water may performed to obtain a solid polyphenol-containing composition. Extracted water-soluble flavors and polyphenols may be then recombined with the above-described extracts from the solid phase to enhance the overall yield of aromas and polyphenolic compounds.

In a second embodiment, the present invention relates to a method of processing cocoa cell cultures, comprising the steps of: a2) incubating a cocoa bean explant in a liquid medium comprising a cell wall disaggregating enzyme, preferably pectinase, to obtain free cocoa bean cells in suspension; b2) subjecting the cocoa bean cells obtained in step a2) to an adaptation step by transferring the same to a liquid medium without the cell wall disaggregating enzyme; c) growing cocoa bean cells in the suspension cell culture for a time and under conditions sufficient to produce a biomass comprising secondary metabolites; and d) separating the biomass from the suspension cell culture to provide *in vitro*-manufactured cocoa powder having an acetic acid content of 200 mg per kg of dry weight or less, and optionally further processing cocoa powder to provide cocoa aroma or a polyphenolic extract; wherein step c) is carried out in a bioreactor selected from a stirred tank bioreactor, an orbitally shaken bioreactor, a bubble column bioreactor or an air-lift bioreactor, preferably a bubble column bioreactor or an air-lift bioreactor.

Accordingly, the method according to the second embodiment is understood to correspond to the method of the first embodiment, with the exception that the steps of callus growth, selection and inoculation according to steps a1) and b1) are replaced by a step a2) of incubating a cocoa bean explant in a liquid medium comprising a cell wall disaggregating enzyme to obtain free cocoa bean cells in suspension, and a step b2) of subjecting the cocoa bean cells obtained in step a2) to an adaptation step by transferring the same to a liquid medium without the cell wall disaggregating enzyme. By circumventing the induction and mass propagation of callus cultures, the overall process of biomass production may be accelerated.

In step a2), an explant of preferably mature cocoa beans is subjected to incubation in a liquid medium (e.g., MS medium, WPM medium, DKW basal medium, Gamborg B5 medium or the like, as well as modifications thereof), to which a cell wall disaggregating enzyme (such as pectinase (pectin methylesterase), for example) is added to disaggregate the cells. Incubation is preferably performed in darkness at temperatures of 20 ± 10 °C.

In terms of a favourable balance of cell viability and effectiveness of the cell disaggregation, the concentration of cell wall disaggregating enzyme in the liquid incubation medium is preferably 0.001 to 0.5 % by weight of the total weight of the incubation medium, further preferably between 0.05 and 0.2 % by weight.

It will be understood that the preferred features of the first embodiment may be freely combined with those of the second embodiment unless they are mutually exclusive.

### In Vitro-Manufactured Extracts and Uses

In a third embodiment, the present invention relates to selected from any of the liquid phase, cocoa powder, cocoa aroma or the polyphenolic extract obtained by the method according to any of the first or the second embodiments.

As it will be immediately apparent to the skilled artisan, the cocoa powder produced by plant cell cultivation according to the present invention fundamentally differs from traditionally manufactured cocoa powder in various ways.

In conventional production of cocoa powder, cocoa beans are initially harvested, fermented and dried. During natural cocoa fermentation, yeasts ferment the glucose of the cocoa pulp into ethanol, perform pectinolysis and produce flavour compounds, lactic acid bacteria ferment the glucose, fructose and citric acid of the cocoa pulp into lactic acid, acetic acid, mannitol and pyruvate, acetic acid bacteria oxidize the ethanol into acetic acid. The thus produced acetic acid penetrates into the bean cotyledons and ultimately inhibits microbial activities. Although it has been found that acetic acid may have a favorable effect on the development of chocolate flavor (see V.C.Quesnel, Proceedings of "1957 Cocoa Conference", pages 150-155), its presence in the beans after the fermentation process is problematic, as it may lead to unpleasantly sour, pungent and bitter flavors in the final cocoa products and additionally tends to mask desirable aromas perceived as pleasant. In order to reduce the concentration of acetic acid in the cocoa material, cocoa beans are typically subjected to a treatment with alkaline solution (in the so-called Dutch process) and/or high roasting temperatures (typically up to 180°C), which, however, inevitably leads to a degradation of antioxidants and vitamins. In general, the process of conching is generally also conducted in a manner which aims at not only lowering the viscosity of the chocolate product but also as a further step for flavor development and removal of undesired flavor artifacts which originate from acidic species in the beans. As it involves maintaining the product at an elevated temperature for a relatively long time, it is both energy intensive and inevitably leads to the destruction of cellular compartments of the raw cocoa materials due to high mechanical loads, shear stresses and/or high heat.

Advantageously, the *in vitro*-manufactured cocoa powder according to the present invention overcomes the above disadvantages as it was shown to exhibit an acetic acid content of 200 mg per kg of dry weight or less, usually less than 100 mg per kg of dry weight, such as 50 mg per kg of dry weight or less, 20 mg per kg of dry weight or less, 10 mg per kg of dry weight or less, or even 1 mg per kg of dry weight or less. In embodiments, the cocoa powder according to the present invention only contains trace amounts of acetic acid (0.1 mg per kg of dry weight or less) or no acetic acid at all. Accordingly, harsh roasting conditions and/or a prolonged conching duration which may negatively affect the yield of heat-sensitive components may be avoided. At the same time, it has been surprisingly found that the absence of acetic acid does not impair the development of complex and characteristic cocoa flavours.

Compared to conventional cocoa powder, the *in vitro*-manufactured cocoa powder of the present invention has been shown to exhibit significantly lower contents of dimethyl trisulfide, which is characterized by a foul odor. Typically, the content of dimethyl trisulfide is below 5 µg/kg of dry weight, such as 4 µg/kg or less, preferably 3 µg/kg or less. On the other hand, the *in vitro*-manufactured cocoa powder of the present invention advantageously exhibits high concentration of pleasant fruity aroma compounds, such as furaneol (4-hydroxy-2,5-dimethyl-3-furanone). In preferred embodiments, the content of furaneol is at least 5 mg/kg, more preferably 7 mg/kg or higher, further preferably 9 mg/kg or higher, based on the total dry weight of the *in vitro*-manufactured cocoa powder.

In a preferred embodiment, the cocoa powder has a (-)-Epicatechin content of at least 4 g/kg dry weight, more preferably at least 10 g/kg dry weight, especially preferably at least 15 g/kg dry weight; a Procyanidin B2 content of at least 3 g/kg dry weight, more preferably at least 4 g/kg dry weight, especially preferably at least 7 g/kg dry weight, further preferably at least 10 g/kg dry weight; a Procyanidin C1 content of at least 2 g/kg dry weight, more preferably at least 4 g/kg dry weight, especially preferably at least 7 g/kg dry weight; and/or a Cinnamtannin A2 content of at least 1 g/kg dry weight, more preferably at least 2 g/kg dry weight, especially preferably at least 3 g/kg dry weight. On the other hand, the content of caffeine is preferably 2 g/kg dry weight or less, typically less than 1 g/kg dry weight; and the content of theobromine is less than 1.5 g/kg dry weight, typically less than 0.8 g/kg dry weight. The respective contents may be determined according to methods known in the art, such as HPLC-MS techniques.

In a preferred embodiment, the *in vitro*-manufactured cocoa powder has a total polyphenol content of at least 5 mg ECE/g of dry matter, more preferably at least 10 mg ECE/g of dry matter, especially preferably at least 20 mg ECE/g of dry matter. The total polyphenol content may be determined by common spectrophotometric methods known to the skilled artisan. For example, the total polyphenol content may be determined by using a Folin-Ciocalteu assay with (-)-epicatechin as standard, in accordance with the method disclosed in Folin-Ciocalteu index, Off. J. Eur. Communities 1990, 41, 178-179, and Cooper et al., J. Agric. Food Chem 2008, 56, 260-265.

*In vitro*-manufactured cocoa powder according to the present invention exhibits very low fat contents, usually less than 1 wt.-% of the total solid weight, typically less than 0.5 wt.-%, preferably less than 0.1 wt.-%. Accordingly, the cocoa powder may be advantageously used for low calorie products and for bakery applications, wherein fat blooming is to be avoided, for example, without necessitating a defatting treatment which typically involves a reduction of nutritionally valuable components.

In a fourth embodiment, the present invention relates to the use of any of the *in vitro-*manufactured extracts according to the third embodiment as food, food additive or in cosmetic or pharmaceutical preparations.

Compositions containing the polyphenolic extract according to the present invention are useful for a variety of applications, such as pharmaceutical products, food supplements, and beverage compositions, and can be prepared and administered in suitable dosage forms according to methods known in the art.

The cocoa aroma according to the present invention may be used to enhance the sensory properties in a wide range of food products.

The *in vitro*-manufactured cocoa powder according to the present invention may be used for the same purposes as conventional cocoa powder, while providing the benefits of favourably high antioxidant contents and a characteristic and complex cocoa flavour.

In preferred embodiments, the cocoa powder according to the present invention is used to manufacture chocolate or chocolate-products, as will be further described below.

However, the cocoa powder may also be used for the preparation of frozen desserts (e.g. chocolate-flavored ice cream, frozen yoghurts, sherbets and sorbets). For this purpose, a mixture comprising *in vitro*-manufactured cocoa powder and a natural or artificial sweetener (optionally with further additives, such as milk fat, milk solids-non-fat (MSNF), stabilizers, emulsifiers or combinations thereof) may be subjected to pasteurization, homogenisation and freezing steps to obtain the cocoa-flavored frozen dessert.

In addition, the cocoa powder may be used for the preparation of chocolate- or cocoa-flavored beverages. For example, the preparation of instant cocoa powder by instantizing the *in vitro*-manufactured cocoa powder by agglomeration techniques such as e.g. spray drying, steam agglomeration, fluidized bed agglomeration, freeze drying agglomeration, or thermal agglomeration may be envisaged.

### Methods of Manufacturing Chocolate or Chocolate-Like Products

In a fifth embodiment, the present invention relates to a method of manufacturing chocolate or chocolate-like products, comprising the steps of: providing an in vitro-manufactured cocoa powder according to any one of the first and second embodiments; optionally subjecting the cocoa powder to an incubation and/or roasting step; mixing the cocoa powder with cocoa butter and optional additives selected from milk solids, emulsifiers, aroma, sugars and/or cocoa pulp; and conching said mixture.

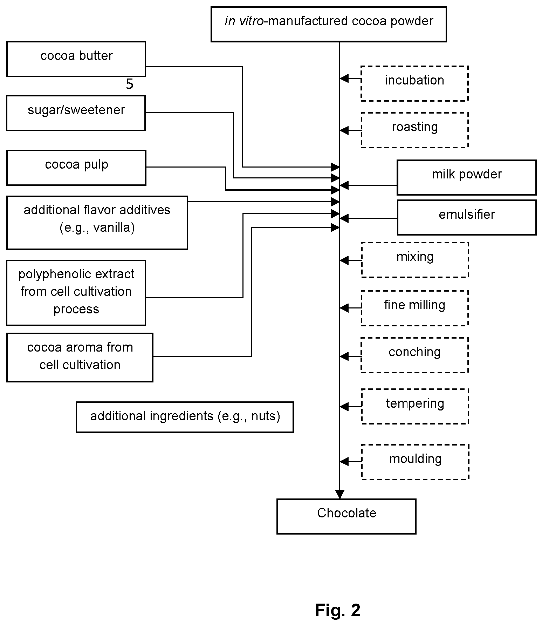

An exemplary method is further illustrated in Fig. 2.

Until recently, the steps of the fermentation, drying and roasting have been considered essential for the formation of the sensory active components. However, new cocoa processing methods show that traditional processing steps such as fermentation or roasting do not necessarily have to be carried out to achieve excellent organoleptic properties.

Incubation represents a simple, reproducible and controllable alternative to microbial (i.e. yeast and bacterial) fermentation, while at the same time a pleasant cocoa flavor and taste profile may be achieved. For this purpose, the cocoa powder is incubated, e.g. at a temperature of between 10 and 70 °C, preferably at a temperature of between 10 and 55°C for a period of between 1 hour and 10 days, preferably between 2 and 168 hours, in an incubation medium under sterile conditions, in order to suppress spontaneous fermentation as effectively as possible.

The incubation medium is not specifically limited and includes aqueous acidic media, as disclosed in US 8,501256 B2, or an aqueous ethanol solution, for example.

In a preferred embodiment, however, the incubation medium is an aqueous ethanol solution. Further preferably, the concentration of ethanol in the incubation medium ranges from 1 to 90 vol.-%, for instance between 1 and 12 vol.-%. In an especially preferred embodiment from the viewpoint of germination inhibition efficiency and processing costs, the concentration of ethanol in the incubation medium is at least 2 vol.-% and less than 7 vol.-%, such as from 2 to 6 vol.%.

In alternative or in addition, the incubation medium may further comprise enzymes known in the art to promote controlled enzyme-catalyzed reactions, which facilitate formation of aroma precursors, such as hydrophilic oligopeptides and hydrophobic free amino acids, for example.

If applied, the incubation may be carried out in a single step or in multiple incubation steps, wherein different incubation conditions (e.g. aerobic or anaerobic) and/or incubation media are employed. As will be known to the skilled artisan, the incubation conditions, such as pH or temperature, may be varied within one single incubation step.

As will be known to the skilled artisan, the incubation step may further encompass one or more mechanical and/or physical treatment steps before or during the incubation, such as stirring, mixing, agitating, an infrared treatment and/or a vacuum treatment, for example.

Roasting conditions are not particularly limited. Gentle drying and roasting procedures involve heating temperatures of between 55 and 120°C, from the viewpoint of reducing the thermal load and preserving the health-inducing components, however, lower temperatures in the range of 55 and 80°C in combination with reduced pressure are employed. This step also enables separation and collection of volatile roasted flavors and other aromatics, which may be recombined with the cocoa aroma extract described above. Roasting may be accomplished in a drum dryer or a preferably continuous turbomixer (as described in EP 0 711 505 A1), for example.

In the next step, the cocoa powder is mixed with cocoa butter and optional additives depending on the desired recipe. Optional additives include emulsifying agents (e.g. lecithin) which reduce viscosity, control sugar crystallization and the flow properties of chocolate, and promote homogeneous mixing; aromas (e.g. vanilla, rum); sugars, sweeteners, cocoa pulp and combinations thereof, among which cocoa pulp is preferred; and additional flavors (e.g. fruit juices, coffee etc.). For the preparation of milk chocolate, milk powder may be added.

At this stage, it is noted that cocoa pulp or the constituents thereof (such as polysaccharides and antioxidants of cocoa pulp) added to the cocoa powder may originate from an independent cultivation process or a co-cultivation of cocoa pulp with the cocoa suspension cells. Moreover, cocoa butter or the lipid constituents thereof (such as palmitic, stearic, oleic, linoleic and linolenic fatty acids) added to the cocoa powder may likewise originate from a separate cultivation or a co-cultivation process. In the latter case, separate cultivation is preferable as it enables specific and selective stimulation to enhance *in vitro* fatty acid production in a controlled manner. Accordingly, it becomes possible to provide a 100% cocoa plant cell-derived chocolate, wherein not only the sweetness and aromatic profile, but also the hardness, texture and fusion properties of the chocolate product may be fine-tuned in the desired manner.

In preferred embodiments, polyphenolic extract and/or cocoa aroma according to the third embodiment are also added to the mixture to provide higher contents of antioxidants and intensified flavor in the final product, respectively.

Subsequently, the mixture is subjected to a conching step. Typically, the temperature of the conche is controlled and varies depending on the different types of chocolate (from around 49 °C for milk chocolate to up to 82 °C for dark chocolate). Traditionally, conching is not only employed to coat solid particles evenly with the fat phase (i.e. cocoa butter) in order to achieve desirable viscosity, flow, and textural properties, but also to remove unwanted acetic, propionic, and butyric acids and thus mellow the flavor of the product. While being to some degree dependent on the temperature, the conching duration in conventional chocolate manufacturing processes thus generally ranges from 16 up to 96 hours in order to achieve good results. Since the method according to the present invention provides a cocoa powder with substantially reduced acid content, the conching duration may be shortened to a period sufficient to only adjust viscosity, flow, and textural properties, which in turn avoids aroma degradation observed at long conching times. In addition, the energy expenditure of the conching process may be substantially reduced. Preferably, the conching duration is less than 16 hours, more preferably less than 12 hours, typically 10 hours or less. The conching equipment is not particularly limited and may be selected from a roller-type conching machines or scraping mixers and agitators.

The thus produced chocolate or chocolate-like products may take any suitable form and may, for example, be packaged and sold as a block or a bar, be filled and may be used as a coating, be used in other confectionery and bakery applications (e.g. as a cake coating or filling, a biscuit coating or filling, a sponge coating or filling or a coating layer for an ice cream). Also, the obtained chocolate or chocolate-like products may optionally have further additives added prior to the final use of the product (e.g. nuts, raisins, dry fruits or the like).

The term "chocolate-like product", as used herein, refers to a product which exhibits similar organoleptic properties as a product falling into the legal definition of a chocolate, but where the labelling as "chocolate" is prohibited under certain legislations in view of a deviation in the type and/or the content range of a component legally defining chocolate, or in view of the fact that an unusual process has been used in the manufacture of the product causing significant changes in the composition or structure of the food or food ingredients which affect the nutritional value and metabolism.

### EXAMPLES

### Example 1 - Cell Culture Chocolate Production based on a Low-Volume Stirred Tank Bioreactor

In an initial step, cocoa beans were isolated from a cocoa pod under aseptic conditions. For this purpose, a cocoa pod was washed and cleaned with alcohol and a rectangular opening was cut with a sterile scalpel (approx. 1.5 cm deep) and opened. Cocoa beans were then extracted, their skin was carefully removed and placed in sterile Petri dishes. The skin-free cocoa beans were subjected to surface sterilization by subsequently treating the same with 70% (v/v) ethanol solution, sterile water, and 5% sodium hypochlorite solution (NaClO) and a final washing step with sterile water.

The surface-sterilized cocoa beans were wounded (cut in half or quartered) and placed in Petri dishes filled with callus growth medium (Murashige and Skoog medium incl. vitamins, modified with 2 mg/L IAA, 2 mg/L IBA and 0.1 mg/L kinetin) and incubated at 29 ° C in the dark. For the subsequent maintenance (subcultivation of beige/brownish calli every 3 weeks) and propagation of the callus cultures, the same medium was employed.

The callus growth medium in liquid form was used to establish suspension cultures. About 1 g of callus (fresh weight) was transferred to a shake flask with 20 mL MS medium and incubated in the dark at a shaking rate of 120 rpm (shaking amplitude 50 mm) and 29 ° C. To remove suspended particles and large aggregates homogenisation by sieving (300 to 500 µm) or sedimentation was performed.

For inoculum production, the cells were expanded into 500 mL shake flasks. The same parameters were used as for maintenance cultivation (29 ° C, 120 rpm, dark culture, 25 mm deflection).

The process was then transferred to an Infors Labfors 5 stirred bioreactor, which was previously sterilized *ex situ* in an autoclave. The bioreactor was equipped with magnetic coupling, enabling aseptic conditions over prolonged cultivation times as well as the installation of different probes. Mixing during cultivation was performed using a 3-blade segment stirrer with a ratio of impeller diameter to internal reactor diameter of 0.58. A dissolved oxygen content of ≥ 30% was regulated by introducing pure oxygen via a sparger below the impeller.

To inoculate the Labfors 5, the biomass was pooled from the shake flask, 388 g (fresh weight) of the filtered biomass was transferred to a sterile vessel, resuspended with cell growth medium (see Table 1) and transferred to the bioreactor. The bioreactor was then filled to 5 L working volume (starting volume) with cell growth medium and cultivation was started.

**Table 1: Cell Growth Medium Composition**

| **Substance** | **Amount per 1L Medium** | **Supplier** |
|---|---|---|
| MS Basal Medium Kit with Vitamins | 1 L Kit | Duchefa |
| Saccharose | 30 g/L | Sigma-Aldrich |
| Indol-3-acetic acid (IAA) | 0.5 mg/L | Sigma |
| 4-(Indol-3-yl)butyric acid (IBA) | 0.5 mg/L | Fluka |
| Zeatin | 0.5 mg/L | Duchefa |
| Kinetin | 0.1 mg/L | Sigma |

The cultivation was carried out under the conditions summarized in Table 2. Samples were taken at intervals of 2 days to determine the conductivity (inversely proportional to the biomass fresh and dry weight) and the sugar and nitrogen consumption (via HPLC). Periodic samples were also taken to visually assess the morphology and color of the cell culture. In addition, the formation of epicatechin, procyanidin B2 and C1, cinnamtannin A2 was investigated via HPLC-MS/MS. Probes for measuring the pH value, conductivity and dissolved oxygen (DO) were implemented in the bioreactor and an external exhaust gas analysis system was connected.

**Table 2: Cultivation Conditions**

| **Parameter** | **Value** |
|---|---|
| Working Volume (Start) | 5 L |
| Working Volume (End) | 10 L |
| Temperature | 29° C |
| Oxygenation Rate (Head) | 0.1 vvm |
| Oxygenation Rate (Sparger) | 0-0.1 vvm |
| Stirring Speed | 50-70 rpm |
| Specific Power Input | 110-150 W/m³ |
| pH after inoculation | 5.5 |
| Dissolved Oxygen Content | 30 % |
| Packed Cell Volume (Start) | 10 % |

The investigation of the biomass with regard to the polyphenols formed in the stirred reactor showed a maximum for epicatechin of 23.75 g·kg⁻¹ dry weight, cinnamtannin A2 of 3.67 g·kg⁻¹ dry weight, procyanidin B2 of g·kg⁻¹ dry weight and procyanidin C1 of g·kg⁻¹ dry weight. No color changes of the biomass were observed during cultivation.

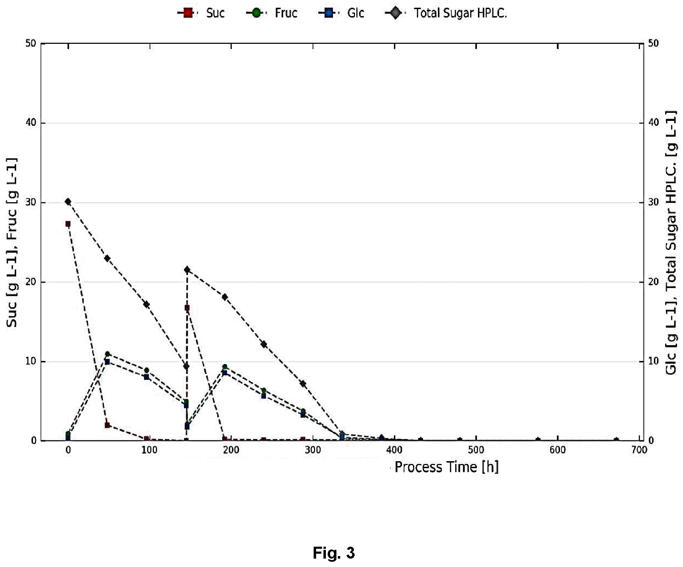

Figure 3 shows the sugar consumption in dependence of cultivation time (Abbreviations: Suc: Sucrose, Fruc: Fructose, Gluc: Glucose, Total Sugar HPLC: Total carbon concentration). After a cultivation time of 146 h, a volume expansion was carried out and the volume was made up to 10 L with fresh medium. The maximum dry biomass weight of 14.67 ± 1.85 g·L⁻¹ was reached on day 28 (672 h) of cultivation.

The biomass was then harvested, separated from the culture broth, rinsed and freeze-dried. The freeze-dried biomass was used as a substitute for conventional cocoa liquor to prepare cell culture chocolate, the complete recipe of which is shown in Table 3. For this purpose the biomass was first roasted in an oven at 135 °C for 10 minutes and then mixed with sugar. Melted cocoa butter was then successively added, while rolling the cocoa mass in a conching step. Upon addition of emulsifying agent (i.e. lecithin), the chocolate was mixed by hand, heated to approx. 32 °C and poured into molds.

**Table 3: Composition of the cell culture chocolate**

| **Constituent** | **Content (in % by weight)** |
|---|---|
| cocoa powder | 25.5 |
| cocoa butter | 44.5 |
| sugar | 29.5 |
| lecithin | 0.5 |

After cooling, the chocolate was vacuum-packed and stored in a refrigerator at 4 °C, before being subjected to a tasting and sensory analysis.

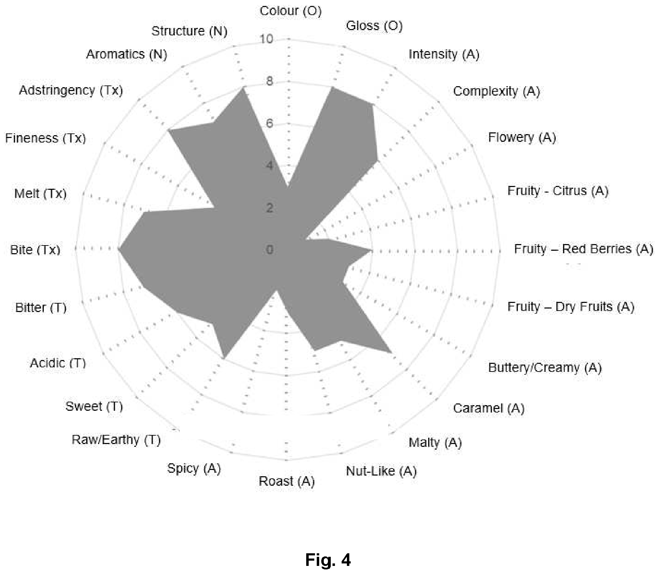

In the sensory evaluation of the chocolate, a sensory profile was created by an experienced panel, rating chocolate on a 10-point scale for 24 attributes in the categories appearance (O), aroma (A), taste (T) and texture (Tx). The results of the sensory analysis are shown in Fig. 4. The chocolate produced with biomass from the stirred reactor was characterized by an intense, fruity and complex aroma and with emphasis on caramel notes.

Accordingly, it was shown that the method of the present invention enables production of chocolate or chocolate-like products with favourable organoleptic properties, including complex and characteristic cocoa flavours. It is further emphasized that the chocolate has been prepared without addition of milk powder, which is known to further mellow the taste profile, and without incorporating any incubation method to mimic flavor development during microbial fermentation. Hence, it is believed that even further optimization of the sensory properties may be easily achieved.

Moreover, the cell cultivation has been carried out in a stirred tank bioreactor, which enables straightforward upscaling to high volume throughput, paving a way to sustainable production of healthy cocoa-based food at a previously inaccessible large scale without the disadvantages of traditional cocoa processing.

### Example 2 - Comparison of Cell Culture Chocolate Produced by Different Bioreactor Types

In a further series of experiments, mass propagation of *T. cacao* suspension cells was carried out in different bioreactor types, i.e. an orbitally shaken bioreactor, an airlift bioreactor, a bubble column bioreactor and stirred tank bioreactors.

The cultivation systems used are listed in Table 4 below. The orbitally shaken bioreactor used was a single-use bioreactor. The stirred, bubble column and airlift bioreactors were reusable systems (with a cultivation vessel made of glass or steel) that were cleaned before and after use and steam sterilized at 121°C for 30 minutes. In the case of the bubble column and airlift bioreactors, additional heated, sterilized exhaust air filters were connected to the reactors due to the increased volume flow of the exhaust air.

**Table 4: Cultivation systems used for mass propagation of T. cacao suspension cells.**

| **Bioreactor Type** | **Device** | **Manufacturer** | **Working Volume [L]** |
|---|---|---|---|
| Orbitally Shaken Bioreactor | Orbshake SB10-X | Kuhner shaker | 10 |
| Stirred Tank Reactor 1 | Labfors 5 | Infors | 10 |
| Stirred Tank Reactor 2 | Biostat D-DCU 100L D100-3 | Sartorius Stedim Biotech | 100 |
| Bubble Column Bioreactor | Biostat D-DCU 100L D100-3 | Sartorius Stedim Biotech | 60 |
| Airlift Bioreactor | Biostat D-DCU 30L D30-3 | Sartorius Stedim Biotech | 24 |

A cocoa suspension culture of clone ICS-45 K1 was used for cultivation. The establishment of a *T. cacao* callus and associated suspension culture was carried out in accordance with Example 1.

A Murashige & Skoog Medium 5 (MS5) was prepared by dissolving MS Medium (incl. vitamins) (4.4053 g·L⁻¹), sucrose (30 g·L⁻¹), indole-3-acetic acid (0.5 mg·L⁻¹), 3-indolebutyric acid 0.5 mg·L⁻¹), kinetin 6-furfurylamino purine (0.1 m g·L⁻¹) and zeatin trans isomer (0.5 mg·L⁻¹) in ultrapure water. After complete dissolution, the pH was adjusted to 5.80 using potassium hydroxide (1 M). Finally, the medium was subjected to sterile filtration with a 0.2 µm filter and stored at 4°C.

For the inoculum production, the cells, starting from the master cell line, were expanded over several passages in 500 mL shake flasks (29° C, 120 rpm, dark culture, 25 mm deflection) using the MS5 medium. In the case of the bubble column and 100 L stirred bioreactor, a wave-mixed bioreactor (10L final volume) was used for inoculum production.

To inoculate the bioreactors, the biomass wass collected from the shake flasks, filtered, resuspended with MS5 medium and transferred to each bioreactor. The bioreactors were then filled with MS5 medium to the appropriate working volume and cultivation was started with the parameters shown in Table 5. In case of the bubble column, airlift and 100 L stirred bioreactor cultivations, the contents of the shake flask used for inoculum production were transferred directly without filtration to reduce the risk of contamination.

**Table 5: Operating parameters for the cultivation stage**

| **Parameter** | **Orbitally Shaken Bioreactor** | **Stirred Tank Bioreactor 1** | **Stirred Tank Bioreactor 2** | **Bubble Column Bioreactor** | **Airlift Bioreactor** |
|---|---|---|---|---|---|
| Working Volume Start | 5 L | 5 L | 100 L | 30 L | 24 L |
| Working Volume End | 10 L | 10 L | 100 L | 60 L | 24 L |
| Cultivation Duration | 28 d | 20 d | 21 d | 22 d | 16 d |
| Temperature | 29°C | 29°C | 29°C | 29°C | 29°C |
| Aeration Rate | 0.1 vvm | 0.1 vvm | 0.03-0.1 vvm | 0.5-1.8 vvm | 0.5-1.4 vvm |
| Mixing Parameter | 50-70 rpm | 30-100 rpm | 100-120 rpm | via gassing | via gassing |
| Dissolved Oxygen | no control | no control | no control | >40 % manual control | >40 % manual control |
| Packed Cell Volume (Inoculation) | 20 % | 20% | 12.5 | 12.5 % | 12.5 % |

As can be seen from Table 5, the working volume (operating volume) was doubled with a single feed of MS5 medium after 7 days in all cultivations except the airlift and 100 L stirred bioreactor cultivation. Where applicable, growth parameters (i.e. pH, p_{O2} (dissolved oxygen), conductivity, PCV (packed cell volume), CFW (wet biomass concentration), CDW (dry biomass concentration), sugar concentration, and cell viability (via staining) were measured in the bioreactors and/or through regular sampling, and the corresponding mixing parameters were modified within the ranges listed in Table 5. After the cultivation, the cells were separated by filtration, rinsed with ultrapure water, shock-frozen for 60 minutes at -25°C, and freeze-dried at a pressure of 0.5 mbar, using a ZIRBUS technology GmbH freeze drying system.

Chocolate samples were produced from each *in vitro*-manufactured cocoa powder using identical manufacturing protocols to obtain the recipes shown in Table 6.

**Table 6: Recipes for dark and milk chocolates.**

| **Ingredient** | **Dark Chocolate** | **Milk Chocolate** |
|---|---|---|
| Cocoa Butter | 45.0 % | 25.0 % |
| In-vitro Cocoa Powder | 25.0 % | 25.0 % |
| Refined Sugar | 29.5 % | 29.5 % |
| Lecithin | 0.5 % | 0.5 % |
| Whole Milk Powder | - | 20.0 % |

For this purpose, the freeze-dried, *in vitro*-manufactured cocoa powder was mixed with a Thermomix for 30 s at maximum power, refined sugar (Schweizer Zucker AG) was added to and mixed with the cocoa powder. Subsequently, deodorized cocoa butter (obtained by Max Felchlin AG; melted at max. 50 °C) was added to the cocoa powder-sugar mixture at a 1:1 weight ratio (based on the cocoa powder), and then mixed in the Thermomix for 30 to 60 s. The thus obtained mixture was then rolled in a three-roll mill first at different pressure settings (10 bar - 22 bar). The rolled mixture was mixed with the remaining amount of melted cocoa butter (max. 50 °C hot), the lecithin (Sunflower lecithin E, obtained from BIC Services B.V.) and whole milk powder LEDOR 26 W obtained from Hochdorf Holding AG (in case of the milk chocolate), heated and melted in a water bath at a maximum temperature of 50 °C, and slowly cooled to 32°C during constant stirring. 1 wt.-% Mycryo^{®} precrystallized cocoa butter (based on the total weight of cocoa butter in each sample) was added to the tempered chocolate and mixed. The liquid chocolate samples were then poured into molds and hardened at 18 °C.

To determine acetic acid concentrations, the cell culture chocolate samples were crushed, weighed (5 g) and extracted with 10 ml of solvent (MTBE Hexane 1:1) after addition of internal standard (propionic acid) until the chocolate was dissolved. The cell culture powder was also extracted after weighing (5 g) in 40 ml solvent after adding the internal standard. Thereafter, the samples were centrifuged, filtered and analyzed via GC-FID. The results of the measurements are shown in Table 10.

**Table 10: Acetic acid concentrations in in vitro-manufactured cocoa powder and cell culture chocolate**

| **Analyte** | **Chocolate Type** | **Reactor used for Manufacturing** | **Acetic Acid (mg/kg)** |
|---|---|---|---|
| Cell Culture Cocoa Powder | - | Stirred Tank Bioreactor 1 | 8.70 |
| | | Orbitally Shaken Bioreactor | 6.18 |
| | | Stirred Tank Bioreactor 2 | 3.32 |
| | | Bubble Column Reactor | 4.56 |
| | | Airlift Bioreactor | 11.22 |
| Chocolate | dark | Stirred Tank Bioreactor 1 | 32.02 |
| | dark | Orbitally Shaken Bioreactor | 6.23 |
| | dark | Stirred Tank Bioreactor 2 | 6.28 |
| | milk | Stirred Tank Bioreactor 2 | 3.10 |
| | dark | Bubble Column Reactor | 13.95 |
| | dark | Airlift Bioreactor | 12.28 |

As is shown in Table 10, the levels of acetic acid determined in the cell culture cocoa powders were lower than the concentrations determined in the chocolate samples, which indicates that significant amounts of acetic acid in the final chocolate products originate from the added cocoa butter. However, acetic acid concentrations in the cell culture chocolate samples are still significantly lower than in the traditionally manufactured industrial chocolate (~250 mg/kg, according to Seyfried et al., J. Agric. Food Chem. 2019, 67, 5827-5837) and "bean-to-bar" chocolates processed under mild heat (> 1000 mg/kg, according to L. Ullrich et al., J. Agric. Food Chem. 2022, 70, 42, 13730-13740).

The cell culture chocolate samples were further analyzed quantitatively on the basis of selected key compounds. Analyte samples were prepared according to A. André et al., Heliyon 2022, 8(9), e10770. The determination of selected key cocoa flavors in the samples was carried out in accordance to L. Ullrich et al., J. Agric. Food Chem. 2022, 70, 42, 13730-13740 using one- or two-dimensional GC-MS.

The results of the aroma key compound analysis carried out on the basis of dark chocolates prepared by using the *in-vitro-*manufactured cocoa powders are shown in Table 11.

**Table 11: Mean concentrations of selected cocoa flavoring substances in µg/kg (n=2; except for samples marked with *, n=1; nd: not detectable)**

| **Analyte** | **Concentration [µg/kg]** | | |
|---|---|---|---|
| | **Airlift Bioreactor** | **Bubble Column Bioreactor** | **Stirred Tank Bioreactor 1** |
| 2-ethyl-3,5-dimethylpyrazine | 70 | 55 | 938 |
| 2-methylbutanoic acid | 197 | 134 | 493 |
| 3-methylbutanoic acid | 406 | 375 | 807 |
| 2-phenylethan-1-ol | 40.02 | 32.46 | 24.68 |
| phenylacetic acid | 3645 | 2379 | 260 |
| 2,3,5-trimethylpyrazine | 72 | 74 | 242 |
| 4-hydroxy-2,5-dimethyl-3-furanone | 9715 | 21419 | 26477 |
| dimethyl trisulfide | 1.90 | 0.42 | 1.29 |
| linalool | 12.46 | 8.39 | 4.95 |
| 2-methoxyphenol | 0.69 | 0.42 | 0.13 |
| γ-nonalactone | 69 | 82 | 118 |
| 4-methylphenol | 0.009 | 0.008 | 0.008 |
| phenylacetaldehyde | 1352 | nd | nd |
| δ-decenolactone | 218 | 230 | 182* |
| 3-methylbutyl acetate | 0.22 | 0.23 | 2.40 |
| 3-methylbutanal | 12.37 | 35.08* | 83* |
| 2-methylbutanal | 30.18 | 85* | 154* |
| 3-hydroxy-4,5-dimethylfuran-2(5H)-one | 4.09 | 3.56 | 20.27 |

Taking into account that cocoa butter does not substantially alter the odorant profile, the results indicate that key odorants previously identified as cocoa odor constituents are formed abundantly in the *in vitro*-manufactured cocoa powders. Furthermore, the results also show that flavor profiles may be fine-tuned depending on the bioreactor selection and operating conditions.

In comparison with traditionally manufactured chocolate (cf. L. Ullrich et al., J. Agric. Food Chem. 2022, 70, 42, 13730-13740), the analyzed chocolate samples contained lower concentrations of acidic components (i.e. 2-methylbutanoic acid, 3-methylbutanoic acid and phenylacetic acid) and substantially lower concentrations of dimethyl trisulfide, to which a foul odor is attributed. On the other hand, the cell culture chocolate samples exhibited substantially higher contents of furaneol (4-hydroxy-2,5-dimethyl-3-furanone), a key odorant of strawberries and pineapples with fresh and fruity attributes.

Fat contents of freeze-dried cell culture cocoa powders were determined with a SMART6^{™} Microwave + Infrared Moisture & Solids Analyzer (CEM) as a double determination. As a result, no or very low fat contents (< 1% by weight of total dry mass) were determined in the cell culture cocoa powders.

Total polyphenol contents of the cocoa powder and chocolate samples were determined by using a Folin-Ciocalteu assay with (-)-epicatechin as standard, in accordance with the method disclosed in Folin-Ciocalteu index, Off. J. Eur. Communities 1990, 41, 178-179, and Cooper et al., J. Agric. Food Chem 2008, 56, 260-265. Contents of epicatechin, procyanidin B2 and procyanidin C1 were determined via HPLC-UV with an Agilent 1260 chromatographic system. Results of the measurements are shown in Table 12.

**Table 12: Total contents of polyphenols and individual polyphenol contents**

| **Sample** | **Chocolate** | **Reactor Type** | **Total Polyphenol Content (mg ECE-equivalents/100g sample)** | **Epicatechin Content (mg/100g sample)** | **Procyanidin B2 Content (mg/100g sample)** | **Procyanidin C1 Content (mg/100g sample)** |
|---|---|---|---|---|---|---|
| Cell Culture Cocoa Powder | - | Stirred Tank Bioreactor 1 (n=2) | 1487.96 ± 93.27 | 37.99 ± 2.57 | 20.35 ± 2.27 | 10.92 ± 0.01 |
| | | Stirred Tank Bioreactor 2 (n=2) | 2144.52 ± 57.04 | 8.54 ± 0.28 | 12.76 ± 0.91 | 3.28 ± 0.02 |
| | | Oribitally Shaken Bioreactor (n=2) | 2207.85 ± 92.62 | 76.97 ± 6.84 | 44.89 ± 1.58 | 31.61 ± 1.92 |
| | | Airlift Bioreactor (n=2) | 2151.62 ± 133.79 | 326.18 ± 30.88 | 99.22 ± 6.68 | 72.39 ± 12.88 |
| | | Bubble Column Reactor(n=2) | 2676.37 ± 73.32 | 94.63 ± 7.19 | 26.84 ± 0.45 | 23.81 ± 2.20 |
| Chocolate | Dark | Stirred Tank Bioreactor 1 (n=3) | 983.59 ± 19.61 | 1.46 ± 0.10 | 9.54 ± 0.78 | < 4.2 |
| | Dark | Stirred Tank Bioreactor 2 (n=3) | 1269.46 ± 43.70 | 2.46 ± 1.06 | 8.73 ± 1.37 | < 4.2 |
| | Milk | Stirred Tank Bioreactor 2 (n=1) | 900.53 ± 73.36 | 2.92 | 11.31 | 5.36 |
| | Dark | Oribitally Shaken Bioreactor (n=3) | 1623.49 ± 56.62 | 46.90 ± 2.43 | 28.26 ± 1.91 | 14.89 ± 0.61 |
| | Dark | Airlift Bioreactor (n=3) | 1341.68 ± 165.25 | 107.54 ± 0.58 | 54.93 ± 1.89 | 23.72 ± 0.65 |
| | Dark | Bubble Column Reactor (n=3) | 1608.25 ± 94.28 | 103.91 ± 3.70 | 52.70 ± 2.83 | 22.40 ± 0.35 |

Compared to cocoa powders obtained from fermented cocoa beans, higher levels of total polyphenols were identified in the cell culture cocoa powders. In the chocolate samples, the levels of the individual polyphenols and the total polyphenol content were at least comparable to the levels found in dark chocolate (cf. L. Ullrich et al., J. Agric. Food Chem. 2022, 70, 42, 13730-13740)

The cell culture-based chocolate samples were further analyzed in a consensus profile test in accordance with DIN EN ISO 13299. As part of the sensory analysis, a total of 5 trained individuals from a specialist panel tested a total of 6 chocolate samples, using an 11-part scale (from 0 - 10) to define the intensity of each attribute. A total of 23 attributes were evaluated in the four categories of appearance, texture, basic taste and aroma.

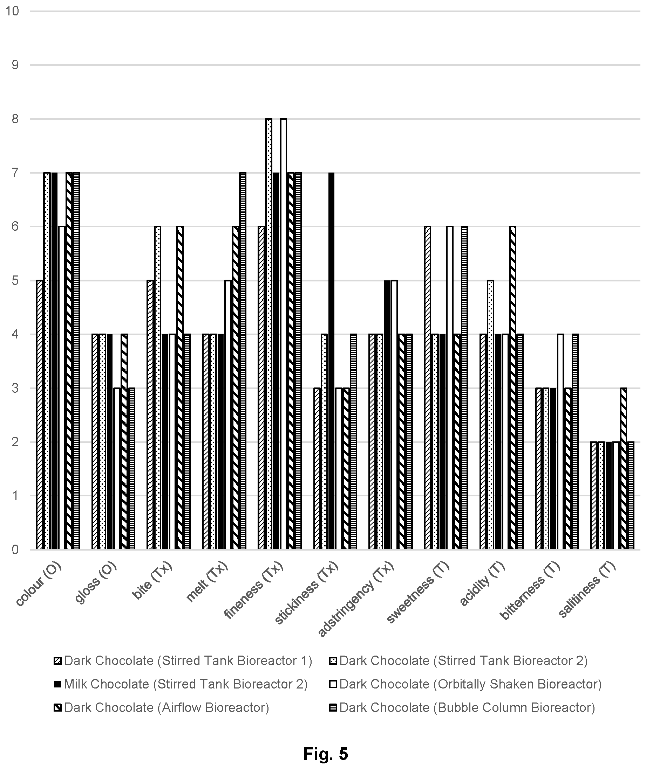

The individual characteristics of the chocolate samples are shown in Figure 5, identifying the sensory properties with respect to color (light to dark), gloss (matte to glossy), bite (soft to hard), melt (slow to fast), fineness (sandy to fine) and stickiness (little to pronounced). In addition, the intensity characteristics of the four basic tastes sweetness, acidity, bitterness and saltiness are listed, from slightly to strongly pronounced.

The individual chocolate samples differ from each other in the optics category, particularly in terms of colour. In terms of texture, there are sometimes significant product differences in terms of melting behavior, fineness and stickiness. For the basic flavors, all seven chocolate samples show a low level of bitterness. The sweetness and acidity are perceived somewhat more intensely than the bitterness, with the latter being associated in particular with fruit acid. A slight saltiness was also detected in all samples.

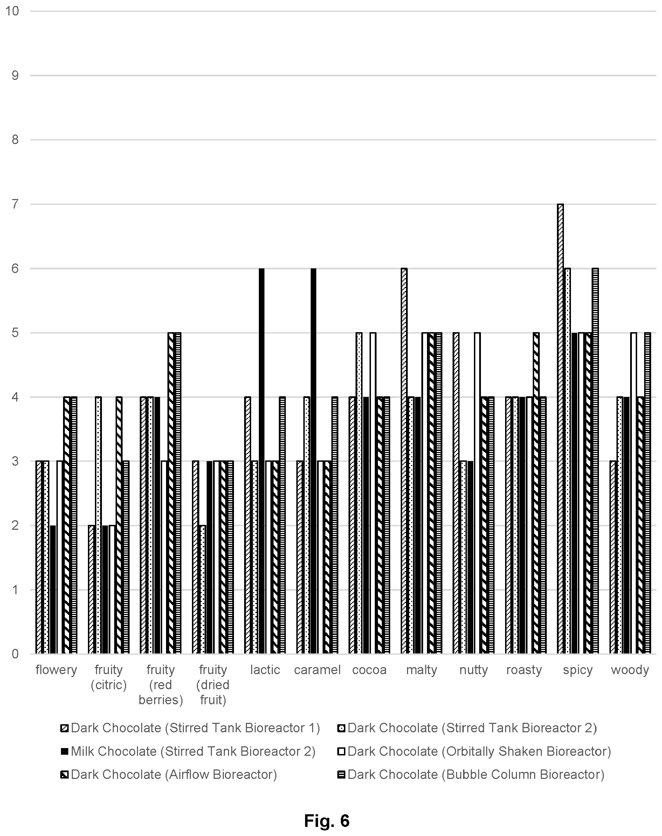

In Figure 6, product profiles with a focus on individual aroma categories are listed. Overall, it is shown that that all chocolate samples are characterized by a complex, characteristic cocoa aroma (retronasal). Some aroma categories such as floral and fruity notes are perceived more subtly (with the exception of Airlift and Bubble Column chocolate samples), while spicy (like liquorice) and malty notes in particular are more clearly perceptible (especially in the Stirred Tank Bioreactor 1 sample). As expected, the lactic notes reminiscent of caramel were more pronounced in the milk chocolate sample.

Overall, the results of the analysis in Example 2 not only demonstrate that the *in vitro*-manufactured cocoa powders not only enable production of cell culture-based chocolate with pleasant and complex flavor profiles, but that the use of stirred tank reactors, airlift bioreactors, orbitally shaken reactors, and bubble column reactors enable an upscaling of the cell production without compromising cell yield, growth rates and culture stability while simultaneously allowing for a fine-tuning of the expression of secondary metabolites.

Once given the above disclosure, many other features, modifications, and improvements will become apparent to the skilled artisan.

## Claims

1. Method of processing cocoa plant cell cultures, comprising the steps of:
a1) inducing callus from a cocoa bean on callus induction medium and propagating the callus on callus growth medium;
b1) selecting a callus clone from the grown callus culture and initiating a suspension cell culture comprising dedifferentiated cells by inoculating the selected callus clone into a suspension cell culture medium;
c) growing cocoa suspension cells for a time and under conditions sufficient to produce a biomass comprising secondary metabolites; and
d) separating the biomass from the suspension cell culture to provide a liquid phase and *in vitro*-manufactured cocoa powder having an acetic acid content of 200 mg per kg of dry weight or less, and optionally further processing said cocoa powder to provide cocoa aroma or a polyphenolic extract;
wherein step c) is carried out in a bioreactor selected from a stirred tank bioreactor, an orbitally shaken bioreactor, a bubble column bioreactor or an air-lift bioreactor, preferably a bubble column bioreactor or an air-lift bioreactor.

2. Method of processing cocoa plant cell cultures, comprising the steps of:
a2) incubating a cocoa bean explant in a liquid medium comprising a cell wall disaggregating enzyme, preferably pectinase, to obtain free cocoa bean cells in suspension;
b2) subjecting the cocoa bean cells obtained in step a2) to an adaptation step by transferring the same to a liquid medium without the cell wall disaggregating enzyme;
c) growing cocoa bean cells in the suspension cell culture for a time and under conditions sufficient to produce a biomass comprising secondary metabolites; and
d) separating the biomass from the suspension cell culture to provide *in vitro-*manufactured cocoa powder having an acetic acid content of 200 mg per kg of dry weight or less, and optionally further processing cocoa powder to provide cocoa aroma or a polyphenolic extract;
wherein step c) is carried out in a bioreactor selected from a stirred tank bioreactor, an orbitally shaken bioreactor, a bubble column bioreactor or an air-lift bioreactor, preferably a bubble column bioreactor or an air-lift bioreactor.

3. The method according to any of claims 1 or 2, wherein the bioreactor has a volume of at least 0.05 m³, preferably at least 0.1 m³, more preferably at least 1 m³, especially preferably at least 5 m³, such as 10 m³ or more, or 100 m³ or more.

4. The method according to any of claims 1 to 3, wherein hydrodynamic parameters and aeration conditions of the bioreactor are determined based on the concentration of target secondary metabolites present in the cocoa powder.

5. The method according to claim 4, wherein the target secondary metabolites comprise one or more selected from the group of polyphenols, vitamins and aromatic compounds; or wherein the target secondary metabolites comprise one or more selected from the group of cinnamtannins, procyanidins, epicatechin, vitamin A, vitamin B1, vitamin B2, vitamin B3, vitamin C, vitamin E, 1-methyl-1H-pyrrole, ethyl-2-methyl butanoate, 3-methylbutyl acetate, 2-heptanone, hexyl acetate, linaool, benzyl alcohol, 2-phenylethanol, ethyl cinnamate, 2-phenethyl acetate, 2-phenyl-2-butenal and ethyloctanoate.

6. The method according to any of claims 4 or 5, wherein the hydrodynamic parameters and aeration conditions include mixing time, specific power input and/or volumetric oxygen transfer coefficient, the specific power input being adjusted within a range of 100 to 800 W/m³, and the volumetric oxygen transfer coefficient being adjusted within a range of 0.3 to 80 h⁻¹.

7. The method according to any one of claims 1 to 6, wherein the suspension cells are not genetically modified.

8. The method according to any one of claims 1 to 7, wherein the suspension cell culture medium comprises a plurality of mineral salts and optionally plant hormones and/or sucrose.

9. The method according to any one of claims 1 to 8, wherein step c) comprises co-cultivating cocoa suspension cells with microorganisms, the microorganisms being preferably selected from one or more species from the group of yeasts, lactic acid bacteria and/or acetic acid bacteria, preferably from one or more species from the group of lactic acid bacteria and/or acetic acid bacteria.

10. The method according to any one of claims 1 to 9, wherein step c) comprises the addition of enzymes, phytohormones or biosynthetic precursors; and/or wherein step c) comprises an elicitation step comprising the addition of one or more abiotic or biotic elicitors to promote biosynthesis of the secondary metabolites.

11. The method according to any one of claims 1 to 10, wherein in step c), growth of the biomass is enhanced by exposing the suspension cell culture to artificial light having a wavelength in the range of 300 to 700 nm and with an intensity sufficient to promote biomass growth and/or by removal of a fraction of the liquid medium during the growth phase.

12. *In vitro*-manufactured extract selected from any of the liquid phase, cocoa powder, cocoa aroma or the polyphenolic extract obtained by the method according to any one of claims 1 to 11.

13. Use of the *in vitro*-manufactured extract according to claim 12 as food, food additive or in cosmetic or pharmaceutical preparations.

14. Method of manufacturing chocolate or chocolate-like products, comprising the steps of:
providing an *in vitro*-manufactured cocoa powder according to any one of claims 1 to 11;
optionally subjecting the cocoa powder to an incubation and/or roasting step;
mixing the cocoa powder with cocoa butter, milk solids and optional additives selected from emulsifiers, aroma, sugars and/or cocoa pulp; and
conching said mixture.

15. Chocolate or chocolate-like products obtained by the method according to claim 14.
